# EUROPEAN PATENT APPLICATION

(11) **EP 4 534 698 A1**
(43) Date of publication of application: **09.04.2025**
(21) Application number: 23866696.0
(22) Date of filing: 30.10.2023
(51) Int. Cl.: C12Q 1/6886, C12Q 1/6851, C12N 15/11

(54) **LIVER-CANCER-RELATED SERUM MICRORNA MARKER AND NEW METHOD FOR DIAGNOSING LIVER CANCER**

(30) Priority: 14.08.2023 CN 202311016224
(71) Applicant: Qingdao Ruiside Medical Laboratory Co., Ltd., Qingdao, Shandong 266000 (CN)
(72) Inventor: CHEN, Mengmeng, Shandong 266000 (CN); ZHANG, Bingqiang, Shandong 266000 (CN); LUAN, Yansong, Shandong 266000 (CN); ZHOU, Yang, Shandong 266000 (CN); HAN, Lihui, Shandong 266000 (CN); LI, Tao, Shandong 266000 (CN); JIA, Xiaoqing, Shandong 266000 (CN); SUN, Yundong, Shandong 266000 (CN); YU, Junmei, Shandong 266000 (CN)
(74) Representative: Cleanthous, Marinos
(86) International application number: PCT/CN2023/127645
(87) International publication number: WO 2025/035595

(57) **Abstract**

The present invention provides liver cancer-associated serum microRNA markers and a new method for diagnosing liver cancer, wherein the liver cancer-associated serum microRNA markers consist of hsa-miR-122, hsa-miR-21, hsa-miR-2115, hsa-miR-221, hsa-miR-27 and hsa-miR-4499; relative expression level of the liver cancer-associated serum microRNA markers in serum is tested using Real-time PCR method, and reagents for the testing contains primers of the liver cancer-associated serum microRNA markers. The present invention can be used to diagnose hepatocellular carcinoma, in particular early hepatocellular carcinoma or to distinguish the serum of at least one patient with hepatocellular carcinoma from the serum of at least one healthy individual, the serum of at least one patient with chronic hepatitis B or the serum of at least one patient with liver cirrhosis.

## Description

### TECHNICAL FIELD

The present invention belongs to the fields of genetic engineering and clinical medicine, and pertains to human liver cancer-associated serum microRNA markers and a new method for diagnosing liver cancer, wherein the serum micro ribonucleic acids (microRNAs) comprise hsa-miR-122, hsa-miR-21, hsa-miR-2115, hsa-miR-221, hsa-miR-27 and hsa-miR-4499.

### BACKGROUND

Hepatocellular carcinoma (HCC) is one of the most common malignant tumors in the world. According to data released by the World Health Organization, there were approximately 19.29 million new cases of cancer and approximately 9.96 million deaths worldwide in 2020. Malignant tumors are one of the most serious threats to human health, and bring a heavy economic burden to the society. The development of liver cancer is a multi-factor, multi-stage process. Globally, chronic HBV and HCV infection are the major risk factors for primary hepatocellular carcinoma (HCC). Especially, chronic HBV infection has a high degree of geographical consistency with incidence of HCC, and is associated with 75% of liver cancer cases worldwide, even reaching 85% in developing countries. Liver cancer is one of the most common malignant tumors in China. According to "Big Data Report on Liver Cancer in China" released in 2018, there are 841,000 new cases of primary liver cancer worldwide every year, and 393,000 new cases in China, accounting for approximately 46.7% of the global total new cases. Chronic hepatitis B virus (HBV) infection is the leading cause of liver cancer in China. Liver cancer mainly includes hepatocellular carcinoma which accounts for 83.9% to 92.3% and intrahepatic cholangiocarcinoma, and about 85% of patients with hepatocellular carcinoma have HBV infection. The 5-year relative survival rate of liver cancer in China is only 12.1%. However, if diagnosed early and treated effectively, a relatively good prognosis can be achieved, especially for small liver cancer within 5 cm, the 5-year survival rate can reach more than 80%. At present, the effectiveness of liver cancer screening is not ideal, and the detection rate and early diagnosis rate are relatively low. Developing early screening methods suitable for a wide range of population is of great significance for the effective prevention and treatment of liver cancer in China.

There is no obvious symptom in the early stage of liver cancer. The traditional early screening method is to test alpha-fetoprotein (AFP), a serological diagnostic marker of liver cancer, in combination with liver B-ultrasound examination for high-risk population. Alpha-fetoprotein, as the most widely used serological marker, plays an important role in the diagnosis and monitoring of liver cancer, but its detection rate for early liver cancer is not satisfactory. At present, both domestic and international studies have proved that there are still deficiencies in the specificity and sensitivity of AFP. Clinically, there are approximately 30% to 40% of AFP-negative patients. The sensitivity of B ultrasound examination is related to the location of the tumor, and there are high skill requirements for examining physicians, which make it difficult to screen and diagnose liver cancer in the early stage.

MicroRNA (miRNA) is a series of evolutionarily conserved single-stranded RNA molecules with a length of 20-25 nucleotides, belonging to endogenous non-coding RNAs. Through incomplete complementary pairing with mRNAs of target genes, miRNA inhibits the translational expression process of proteins or promotes mRNA degradation. The regulation mechanism of miRNA is complex and diverse. By regulating the expression of target genes, miRNA can direct a series of biological mechanisms, such as embryonic growth, cell growth, apoptosis and cell differentiation. As a tumor marker, miRNA has the following five advantages: (1) miRNA is stably expressed and can stably exist in normal human peripheral blood without significant individual differences; (2) miRNA in serum or plasma is not easily degraded when incubated at room temperature for 24 hours or more, subjected to repeated freezing and thawing, over-acidic or over-alkaline conditions; (3) changes in the expression level of miRNA are closely related to the pathological process of malignant tumors; (4) abnormal expression of miRNA in serum can be used as direct evidence for the existence of tumor cells, and has a good biological marker value; and (5) compared to protein biomarkers, miRNA not only has higher testing accuracy, but also is easier to achieve simultaneous testing of multiple components, with obvious advantages. miRNA is involved in many cellular activities in human body, such as proliferation, apoptosis and migration. Moreover, miRNA plays an important role in the proliferation and metastasis of cancer, and can be used as a biomarker for tumorigenesis, tumor development and prognosis.

In previous studies, our company found for the first time that compared with the control group, hsa-miR-2115 and hsa-miR-4499 had significant differences in serum expression levels in patients with liver cancer, regulating the activity of liver cancer cells, and had a certain guiding role in early diagnosis of liver cancer, and had the potential to be used as a marker for early diagnosis of HCC. By combining hsa-miR-122, hsa-miR-21, hsa-miR-2115, hsa-miR-221, hsa-miR-27 and hsa-miR-4499, the diagnostic effect can be further improved, and the diagnostic value can be enhanced ^{[1-4]}. Combined application of multiple miRNA biomarkers can provide rapid, accurate and low-cost early diagnosis for patients with HCC.

### References:

[1] Xu J, Wu C, Che X, et al. Circulating microRNAs, miR-21, miR-122 and miR-223 in patients with hepatocellular carcinoma or chronic hepatitis. [J]. Molecular Carcinogenesis, 2011(2):50.
[2] Zuo D, Chen L, Liu X, et al. Combination of miR-125b and miR-27a enhances sensitivity and specificity of AFP-based diagnosis of hepatocellular carcinoma [J]. Tumor Biology, 2016, 37(5):6539-6549. DOI:10.1007/s13277-015-4545-1.
[3] Wang C, Hann H W, Ye Z, et al. Prospective evidence of a circulating microRNA signature as a non-invasive marker of hepatocellular carcinoma in HBV patients [J]. Oncotarget, 2017, 8(10):-. DOI:10.18632/oncotarget.9429.
[4] Shaker O, Alhelf M, Morcos G, et al. miRNA-101-1 and miRNA-221 expressions and their polymorphisms as biomarkers for early diagnosis of hepatocellular carcinoma [J]. Infection, genetics and evolution: journal of molecular epidemiology and evolutionary genetics in infectious diseases, 2017, 51:173-181.DOI:10.1016/j.meegid.2017.03.030.

### SUMMARY

It is an object of the present invention to provide a group of liver cancer-associated serum microRNA markers and a new method for diagnosing liver cancer, which can further improve the diagnostic effect of liver cancer and improve the diagnostic value.

The technical solution adopted by the present invention is as follows:

The present invention provides liver cancer-associated serum microRNA markers which consist of hsa-miR-122, hsa-miR-21, hsa-miR-2115, hsa-miR-221, hsa-miR-27 and hsa-miR-4499; wherein a sequence of the hsa-miR-122 is tggagtgtgacaatggtgtttg (SEQ ID No.1), a sequence of the hsa-miR-21 is tagcttatcagactgatgttga (SEQ ID No.2), a sequence of the hsa-miR-2115 is catcagaattcatggaggctag (SEQ ID No.3), a sequence of the hsa-miR-221 is agctacattgtctgctgggtttc (SEQ ID No.4), a sequence of the hsa-miR-27 is ttcacagtggctaagttccgc (SEQ ID No.5), and a sequence of the hsa-miR-4499 is gattgctctgcgtgcggaatcgac (SEQ ID No.6).

The present invention provides a new method for diagnosing liver cancer, wherein after sampling and processing the serum to be tested, relative expression level of liver cancer-associated serum microRNA markers in serum is measured using Real-time PCR method; reagents for testing the liver cancer-associated serum microRNA markers hsa-miR-122, hsa-miR-21, hsa-miR-2115, hsa-miR-221, hsa-miR-27 and hsa-miR-4499 contain primers for the markers, and specific composition is as follows: a sequence of a forward primer hsa-miR-122-F of the marker hsa-miR-122 (SEQ ID No.1) is gccgagtggagtgtgacaatg (SEQ ID No.7), a sequence of a forward primer hsa-miR-21-F of the marker hsa-miR-21 (SEQ ID No.2) is tcggcaggtagcttatcagac (SEQ ID No.8); a sequence of a forward primer hsa-miR-2115-F of the marker hsa-miR-2115 (SEQ ID No.3) is catcagaattcatggagget (SEQ ID No.9); a sequence of a forward primer hsa-miR-221-F of the marker hsa-miR-221 (SEQ ID No.4) is gccgagagctacattgtctgc (SEQ ID No.10); a sequence of a forward primer hsa-miR-27-F of the marker hsa-miR-27 (SEQ ID No.5) is gccgagttcacagtggctaag (SEQ ID No.11); a sequence of a forward primer hsa-miR-4499-F of the marker hsa-miR-4499 (SEQ ID No.6) is gattgctctgcgtgcggaatc (SEQ ID No.12); and a sequence of a universal reverse primer (Universal Rp) of the markers is cagtgcagggtccgaggt (SEQ ID No.13).

The present invention provides a new method for diagnosing liver cancer, wherein the liver cancer-associated serum microRNA markers hsa-miR-122, hsa-miR-21, hsa-miR-2115, hsa-miR-221, hsa-miR-27 and hsa-miR-4499 are measured to obtain ΔCt values which are entered into a determination formula:
comprehensive determination value = -1.772+0.097*ΔCt hsa-miR-122 +0.295*ΔCt hsa-miR-21 -1.123*ΔCt hsa-miR-2115 -0.322*ΔCt hsa-miR-221 +0.606*ΔCt hsa-miR-27 +0.057*ΔCt hsa-miR-4499; and the comprehensive determination value ≥ 0 is considered positive and indicates a high risk of liver cancer, and the comprehensive discrimation value < 0 is considered negative and indicates a low risk of liver cancer.

The liver cancer-associated serum microRNA markers of the present invention are selected from more of hsa-miR-122, hsa-miR-21, hsa-miR-2115, hsa-miR-221, hsa-miR-27 and hsa-miR-4499.

To facilitate testing, reagents and tools for determining the liver cancer-associated serum microRNA markers are prepared into a kit containing the reagents that are primers capable of determining expression level of these serum microRNA markers in serum and containing the liver cancer-associated serum microRNA markers described above (hsa-miR-122, hsa-miR-21, hsa-miR-2115, hsa-miR-221, hsa-miR-27 and hsa-miR-4499). The specific implementation is as follows:

A diagnostic kit for human liver cancer comprises primers for the liver cancer-associated serum microRNA markers described above, measuring reagents corresponding to the microRNA markers hsa-miR-122, hsa-miR-21, hsa-miR-2115, hsa-miR-221, hsa-miR-27 and hsa-miR-4499 respectively comprise a forward primer and a reverse primer, and the 6 pairs of primers are specifically as follows:
a sequence of the forward primer hsa-miR-122-F of the marker hsa-miR-122 (SEQ ID No.1) is gccgagtggagtgtgacaatg (SEQ ID No.7), and a sequence of the universal reverse primer (Universal Rp) is cagtgcagggtccgaggt (SEQ ID No.13);
a sequence of the forward primer hsa-miR-21-F of the marker hsa-miR-21 (SEQ ID No.2) is tcggcaggtagcttatcagac (SEQ ID No.8), and the sequence of the universal reverse primer (Universal Rp) is cagtgcagggtccgaggt (SEQ ID No.13);
a sequence of the forward primer hsa-miR-2115-F of the marker hsa-miR-2115 (SEQ ID No.3) iscatcagaattcatggaggct (SEQ ID No.9), and the sequence of the universal reverse primer (Universal Rp) is cagtgcagggtccgaggt (SEQ ID No.13);
a sequence of the forward primer hsa-miR-221-F of the marker hsa-miR-221 (SEQ ID No.4) is gccgagagctacattgtctgc (SEQ ID No.10), and the sequence of the universal reverse primer (Universal Rp) iscagtgcagggtccgaggt (SEQ ID No.13);
a sequence of the forward primer hsa-miR-27-F of the marker hsa-miR-27 (SEQ ID No.5) is gccgagttcacagtggctaag (SEQ ID No.11), and the sequence of the universal reverse primer (Universal Rp) is cagtgcagggtccgaggt (SEQ ID No.13); and
a sequence of the forward primer hsa-miR-4499-F of the marker hsa-miR-4499 (SEQ ID No.6) is gattgctctgcgtgcggaatc (SEQ ID No.12), and the sequence of the universal reverse primer (Universal Rp) is cagtgcagggtccgaggt (SEQ ID No.13).

The reagents other than the primers in the kit can be the reagents commonly used in the corresponding testing techniques in the prior art.

The present inventors collected qualified blood samples according to standard operating procedures (SOP), systematically collected complete basic information and clinical data of the population, and performed the testing using a variety of methods such as RT-PCR, TaqMan miRNA Array and Real-time PCR (TaqMan probe).

Specifically, the experimental methods studied mainly included the following parts:

### I. Selection of study subjects and grouping basis

Group A: control group (n = 345, 150 healthy controls, 150 patients with chronic hepatitis B, 145 patients with liver cirrhosis), no other major systemic diseases; and
Group B: patients with liver cancer (n = 233), no other major systemic diseases.

### II. Blood serum separation and pretreatment

(1) Peripheral blood (2 ml) was drawn into a separating gel blood collection tube and gently mixed well at 180° upside down for 5-6 times. Within 12 hours, the pro-coagulation blood collection tube was centrifuged at 3,000 g for 10min. The resulting supernatant was subsequently transferred to a clean tube and stored at -80°C.
(2) Total RNA was extracted using the miRNeasy Serum/Plasma Advanced Kit following the instructions provided by the manufacturer (Qiagen) and its concentration was quantified using a Qubit 4.0 fluorimeter.

### III. Testing of serum miRNAs expression level using Real-time PCR method

1. cDNA samples were obtained from pre-treated serum by RNA reverse transcription reaction; and the reverse transcription system was formulated as shown in the following table:

| Component | Volume (µL) |
|---|---|
| 10x Poly(A) Polymerase Buffer | 2 |
| E.coli Poly(A) Polymerase (5U/µl) | 0.5 |
| ATP (10mM) | 1 |
| dNTP Mix (10µM) | 1 |
| HiScript II Reverse Transcriptase (200U/µl) | 1 |
| RNase inhibitor (40µM) | 1 |
| RT primer universal (5µM) | 1 |
| RNA | 10 |
| RNase-free ddH₂O | 2.5 |
| Total | 20 |

2. The PCR tube was mixed well repeatedly upside down for 8 times before a brief centrifugation, and placed on ice for 3min.
3. The PCR tube was placed into a gene amplifier for reverse transcription under the following reaction conditions:
42°C, 60min; 85°C, 5min; 4°C, forever.
The resulting reverse transcription product was stored in a 4°C freezer for further pre-amplification.

4. After the reverse transcription, cDNA was pre-amplified using the reaction system as formulated in the following table:

| Component | Volume (µL) |
|---|---|
| Pre-amplification mix (2x) | 12.5 |
| Pre-amplification primer (10x) | 2.5 |
| Reverse transcription product | 2.5 |
| RNase-free ddH₂O | 7.5 |
| Total | 25 |

The reaction conditions for the pre-amplification are shown in the following table:

| Stage | Temperature (°C) | Time |
|---|---|---|
| Holding | 95 | 2min |
| Holding | 55 | 2min |
| Holding | 72 | 2min |
| Cycles (15 times) | 95 | 15s |
| | 60 | 1min |
| Holding (inactivation of enzyme) | 95 | 2min |

After cooling to 4°C, the resulting pre-amplification product was stored at 4°C for the next Real-time PCR reaction.
5. After the pre-amplification product was briefly centrifuged, a total of 75 µl of 0.1x TE (pH8.0) was added for mixing well upside down followed by a brief centrifugation. The pre-amplification product may be directly used in the following Real-time PCR.

The pre-amplification product was subject to Real-time PCR according to the reaction system as formulated in the following table:

| Component | Volume required for one microassay (µL) |
|---|---|
| Universal PCR mix (2x) | 400 |
| Diluted pre-amplification product | 8 |
| RNase-free ddH₂O | 392 |
| Total | 800 |

### 6. Expression levels of miRNAs in serum samples from healthy controls and patients with liver cancer were tested and compared.

Serum miRNAs tested to be differentially expressed in the control group and the patients with liver cancer included hsa-miR-107, hsa-miR-122, hsa-miR-1246, hsa-miR-192, hsa-miR-199, hsa-miR-21, hsa-miR-2115, hsa-miR-221, hsa-miR-223, hsa-miR-26, hsa-miR-27, hsa-miR-4499 and hsa-miR-801.

Copy numbers of these miRNAs were significantly higher in the patients with liver cancer than in the controls.

### IV. Validation of serum miRNAs expression level using Real-time PCR method in a training set

1. Primers were designed for 13 target miRNAs: the primers were designed by PolyA tail PCR method.
2. The Real-time PCR reaction was performed by adding a fluorescent probe. Expression levels of miRNAs were tested and compared in serum samples from healthy controls, patients with chronic hepatitis B, patients with liver cirrhosis and patients with liver cancer (68 healthy controls, 62 patients with chronic hepatitis B, 61 patients with liver cirrhosis, and 101 patients with liver cancer).
3. After the Real-time PCR reaction, there were 6 miRNAs (6miRNA for short) with consistent results, that is, hsa-miR-122, hsa-miR-21, hsa-miR-2115, hsa-miR-221, hsa-miR-27 and hsa-miR-4499.

Therefore, the serum miRNAs of the healthy controls and the patients with liver cancer with differential expression were finally confirmed to include hsa-miR-122, hsa-miR-21, hsa-miR-2115, hsa-miR-221, hsa-miR-27 and hsa-miR-4499.

The above miRNAs with significant expression difference were incorporated into a Logistics regression equation to obtain: Logit (p=HCC)=-1.772+0.097*hsa-miR-122 +0.295*hsa-miR-21 -1.123*hsa-miR-2115 -0.322*hsa-miR-221 +0.606*hsa-miR-27 +0.057*hsa-miR-4499.

### V. Assessment of performance of the above miRNAs combination in a validation set

The performance of the 6miRNA combination was validated in 298 independent serum samples (70 healthy controls, 65 patients with chronic hepatitis B, 63 patients with liver cirrhosis, and 100 patients with liver cancer) to assess ability of the 6miRNA combination to predict liver cancer.

### VI. Method for preparing the diagnostic kit

Based on the above series of experimental results, the present inventors also prepared a microRNA diagnostic kit for human liver cancer, and the diagnostic kit comprised primers and tools for determining testable mature hsa-miR-122, hsa-miR-21, hsa-miR-2115, hsa-miR-221, hsa-miR-27, hsa-miR-4499 in the serum of subjects. The diagnostic kit comprised a batch of serum miRNAs primers, and may also comprise reagents such as Taq enzyme and deoxynucleotide triphosphate.

### BRIEF DESCRIPTION OF THE DRAWINGS

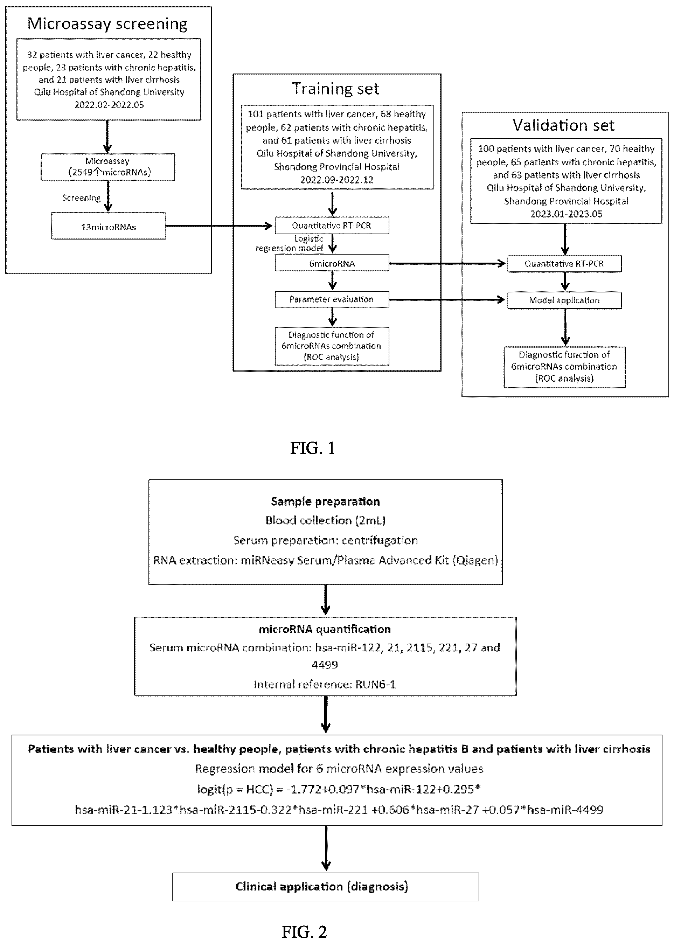

FIG. 1 is a flow chart of an experimental design of screening, training and validation stages of a microRNA combination for identifying at least one target serum of hepatocellular carcinoma, in particularly early hepatocellular carcinoma (BCLC stages 0 and A) according to the present invention.
FIG. 2 is a step diagram of a main method for determining a combination of microRNAs in the blood of patients with hepatocellular carcinoma, in particular with early hepatocellular carcinoma (BCLC stages 0 and stage A) according to the present invention. A control group comprises healthy subjects, subjects with chronic hepatitis B and subjects with liver cirrhosis.

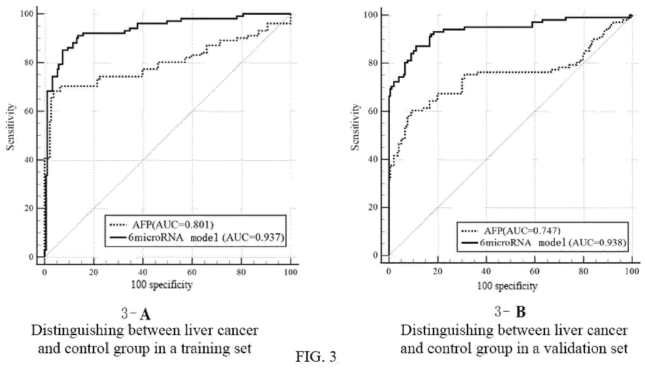

FIG. 3 illustrates a logistic regression model comprising a preferred microRNA combination (hsa-miR-122, hsa-miR-21, hsa-miR-2115, hsa-miR-221, hsa-miR-27 and hsa-miR-4499) for determining at least one target serum of hepatocellular carcinoma according to the present invention;
FIG. 3-A: a ROC plot of the diagnostic value of the microRNA combination in an HCC group compared to a control group in a training set (n=292). Compared with AFP (AUC=0.801), the microRNA combination has significantly higher diagnostic accuracy in distinguishing between the serum from the HCC group and the serum from the control group (AUC=0.937); and
FIG. 3-B: a ROC plot of the diagnostic value of the microRNA combination in the HCC group compared to the control group in a validation set (n=298). Compared with AFP (AUC=0.747), the microRNA combination has significantly higher diagnostic accuracy in distinguishing between the serum from the HCC group and the serum from the control group (AUC=0.938).

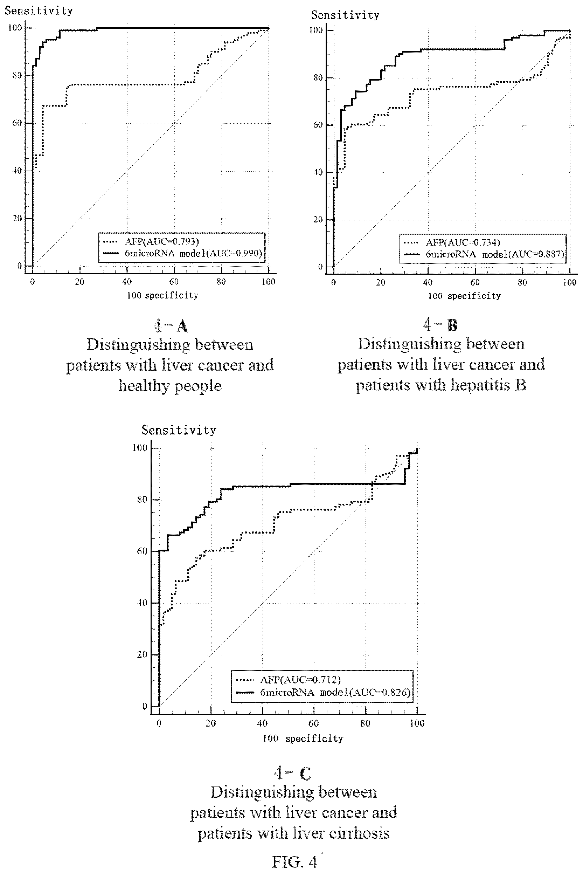

FIG. 4 illustrates a logistic regression model comprising a preferred microRNA combination (hsa-miR-122, hsa-miR-21, hsa-miR-2115, hsa-miR-221, hsa-miR-27 and hsa-miR-4499) for further distinguishing between the serum from the patients with hepatocellular carcinoma and the serum from healthy individuals, patients with chronic hepatitis B or patients with liver cirrhosis according to the present invention;
FIG. 4-A: a ROC plot of the diagnostic value of the microRNA combination in the HCC group compared to the healthy group in a validation set (n=298). Compared with AFP (AUC=0.793), the microRNA combination has significantly higher diagnostic accuracy in distinguishing between the serum from the patients with HCC and the serum from the healthy individuals (AUC=0.990);
FIG. 4-B: a ROC plot of the diagnostic value of the microRNA combination in the HCC group compared to the chronic hepatitis B group in the validation set (n=298). Compared with AFP (AUC=0.734), the microRNA combination has significantly higher diagnostic accuracy in distinguishing between the serum from the patients with HCC and the serum from the patients with chronic hepatitis B (AUC=0.887); and
FIG. 4-C: a ROC plot of the diagnostic value of the microRNA combination in the HCC group compared to the liver hepatitis group in the validation set (n=298). Compared with AFP (AUC=0.712), the microRNA combination has significantly higher diagnostic accuracy in distinguishing between the serum from the patients with HCC and the serum from the patients with liver hepatitis (AUC=0.826).

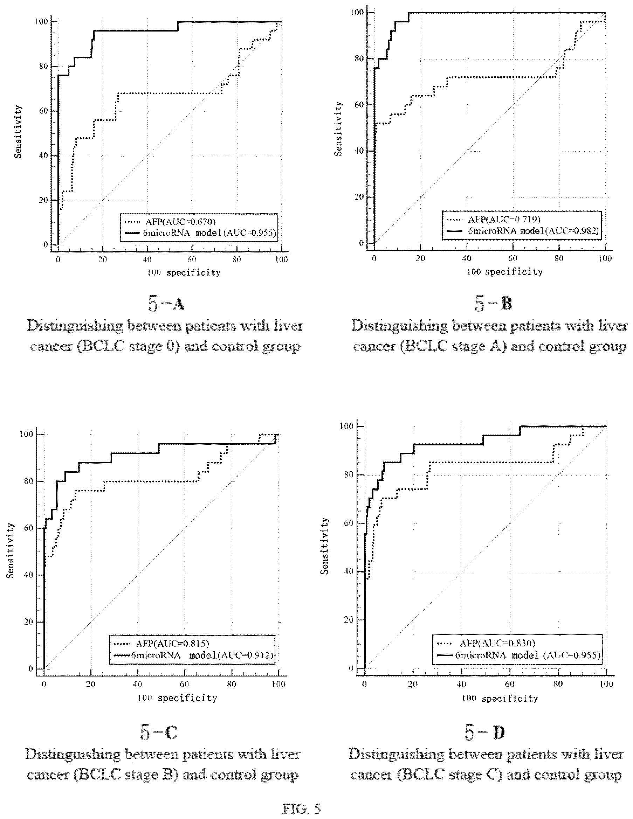

FIG. 5 illustrates a logistic regression model comprising a preferred microRNA combination (hsa-miR-122, hsa-miR-21, hsa-miR-2115, hsa-miR-221, hsa-miR-27 and hsa-miR-4499) for identifying at least one target serum of hepatocellular carcinoma of different BCLC stages according to the present invention;
FIG. 5-A: a ROC plot of the diagnostic value of the microRNA combination in the very early HCC (BCLC 0) compared to the control group. Compared with AFP (AUC=0.670), the microRNA combination has much higher diagnostic accuracy in distinguishing between the serum from the patients with very early HCC and the serum from the control group (AUC=0.955); and
FIG. 5-B: a ROC plot of the diagnostic value of the microRNA combination in the early HCC (BCLC A) compared to the control group. Compared with AFP (AUC=0.719), the microRNA combination has much higher diagnostic accuracy in distinguishing between the serum from the patients with early HCC and the serum from the control group (AUC=0.982);
FIG. 5-C: a ROC plot of the diagnostic value of the microRNA combination in intermediate HCC (BCLC B) compared to the control group. Compared with AFP (AUC=0.815), the microRNA combination has much higher diagnostic accuracy in distinguishing between the serum from the patients with intermediate HCC and the serum from the control group (AUC=0.912); and
FIG. 5-D: a ROC plot of the diagnostic value of the microRNA combination in advanced HCC (BCLC C) compared to the control group. Compared with AFP (AUC=0.830), the microRNA combination has much higher diagnostic accuracy in distinguishing between the serum from the patients with advanced HCC and the serum from the control group (AUC=0.955).

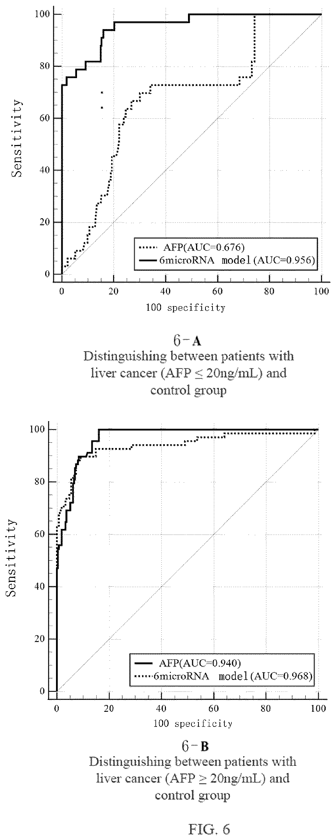

FIG. 6 illustrates a logistic regression model comprising a preferred microRNA combination (hsa-miR-122, hsa-miR-21, hsa-miR-2115, hsa-miR-221, hsa-miR-27 and hsa-miR-4499) for identifying at least one target serum of hepatocellular carcinoma with AFP ≤ 20ng/mL and AFP>20ng/mL according to the present invention;
FIG. 6-A: a ROC plot of the diagnostic value of the microRNA combination in the HCC group with AFP≤20ng/mL compared to the control group. Compared with AFP (AUC=0.676), the microRNA combination has much higher diagnostic accuracy in distinguishing between the serum from the HCC group with AFP≤ 20ng/mL and the serum from the control group (AUC=0.956); and
FIG. 6-B: a ROC plot of the diagnostic value of the microRNA combination in the HCC group with AFP > 20ng/mL compared to the control group. Compared with AFP (AUC=0.940), the microRNA combination has no significant difference in distinguishing between the serum from the HCC group with AFP>20ng/mL and the serum from the control group (AUC=0.968).

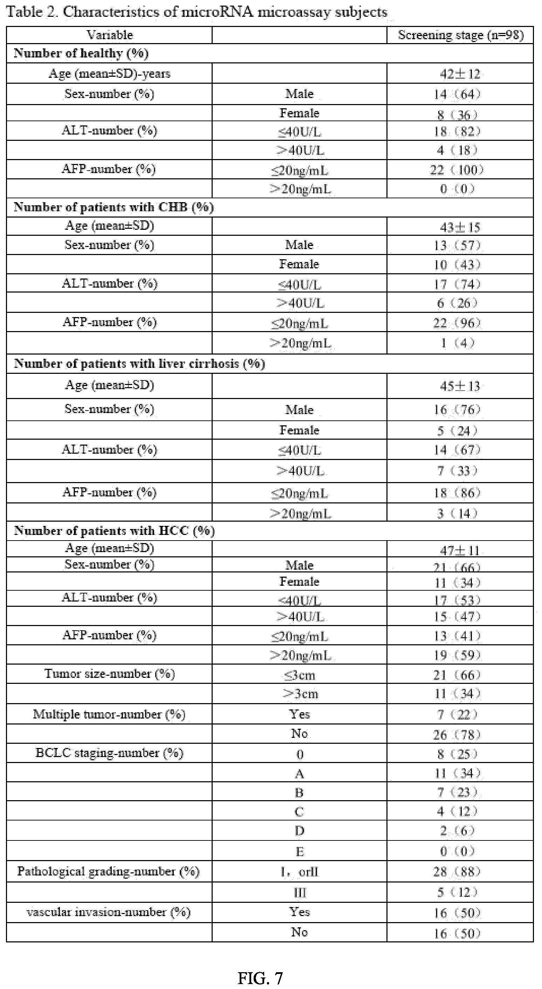

FIG. 7 is Table 2 showing characteristics of microRNA microassay subjects;

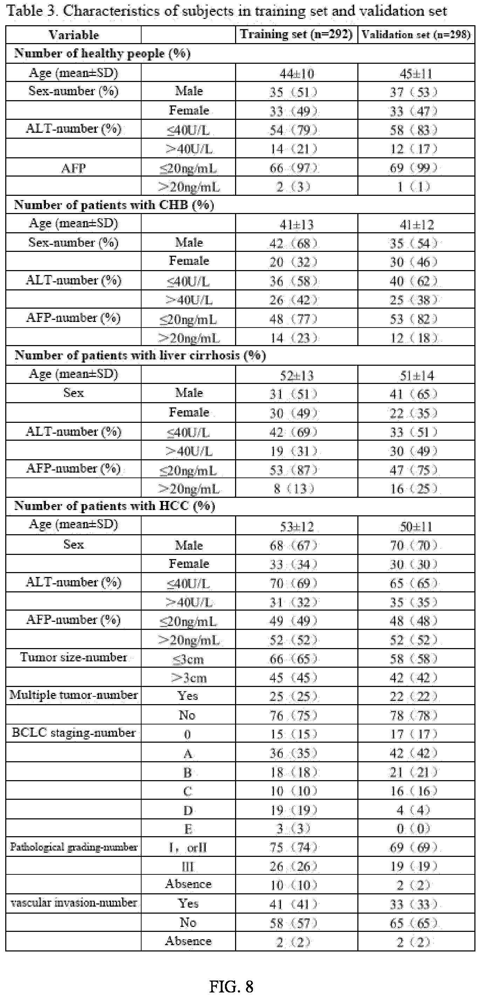

FIG. 8 is Table 3 showing characteristics of subjects in the training set and the validation set;

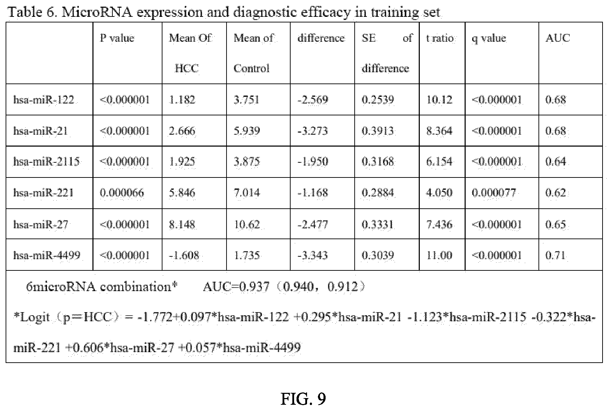

FIG. 9 is Table 6 showing MicroRNA expression and diagnostic efficacy in the training set.

### DESCRIPTION OF THE EMBODIMENTS

The present invention is further illustrated by the following examples.

### Example 1 Selection of study subjects and grouping basis

An experimental design of a screening phase, training phase and validation phase of the microRNA biomarkers according to the present invention is as shown in FIG. 1. The main method steps for identifying serum samples from patients with hepatocellular carcinoma using a recommended combination of serum microRNAs are as shown in FIG. 2.

From January 2022 to May 2023, 688 blood samples meeting the eligibility criteria (Table 1) were collected in advance from Qilu Hospital of Shandong University and Shandong Provincial Hospital. These samples were obtained from 150 healthy donors (HC healthy group), 150 patients with chronic hepatitis B (CHB group), 145 patients with liver cirrhosis after HBV infection (LC liver cirrhosis group), and 233 patients with HCC associated with HBV infection (HCC group). These samples were classification into 3 stages in chronological order (FIG. 1). Clinical manifestations of the patients are summarized in Tables 2 and 3. see FIGS. 7 and 8.

**Table 1 - Eligibility Criteria for Selecting Patients**

| **Inclusion criteria** |
|---|
| 1. Age ≥ 18 years and ≤ 90 years |
| 2. Having ability to give informed consent |
| 3. Met between January 2022 and May 2023 |

| **Healthy donors (healthy group)** |
|---|
| 1. Had a physical examination in the hospital |
| 2. In general health, without malignant tumor |

| **Patients with chronic hepatitis B (CHB group)** |
|---|
| Diagnosed with chronic hepatitis B (i.e., presenting HBsAg+ and HBeAg+, or |

| **Patients with liver cirrhosis after HBV infection (liver cirrhosis group)** |
|---|
| 1. Suffering from HBV infection |
| 2. Diagnosed by two experienced pathologists |
| 3. The diagnosis was supported by two influential reports (B-ultrasound, CT or MRI) |

| **Patients with HCC associated with HBV infection (HCC group)** |
|---|
| 1. Suffering from HBV infection |
| 2. Diagnosed by two experienced pathologists |
| 3. The diagnosis was supported by two influential reports (B-ultrasound, CT or MRI) |
| 4. No preoperative radiotherapy, chemotherapy, hepatic arterial chemoembolization or |

### Example 2 Blood serum separation and pretreatment

Peripheral blood (2 ml) was drawn into a separating gel blood collection tube and gently mixed well at 180° upside down for 5-6 times. Within 12 hours, the pro-coagulation blood collection tube was centrifuged at 3,000 g for 10min. The resulting supernatant was subsequently transferred to a clean tube and stored at -80°C.

Total RNA was extracted using the miRNeasy Serum/Plasma Advanced Kit following the instructions provided by the manufacturer (Qiagen) and its concentration was quantified using a Qubit 4.0 fluorimeter.

### Example 3 MicroRNA microarray analysis of expression differences of miRNAs

MicroRNA microarray analysis was performed to compare the expression levels of microRNA in the HCC group, healthy control group and chronic hepatitis B group and liver cirrhosis group. Serum miRNAs having differential expression in patients with HCC, healthy controls, patients with chronic hepatitis B and patients with liver cirrhosis were screened out, including hsa-miR-107, hsa-miR-122, hsa-miR-1246, hsa-miR-192, hsa-miR-199, hsa-miR-21, hsa-miR-2115, hsa-miR-221, hsa-miR-223, hsa-miR-26, hsa-miR-27, hsa-miR-4499 and hsa-miR-801 (see Table 4 for microRNA sequences).The copy numbers of these miRNAs in patients with HCC were significantly higher than those in healthy controls, patients with chronic hepatitis B and patients with liver cirrhosis.

**Table 4. MicroRNA Sequence Table**

| microRNA | Sequence (5'→ 3') |
|---|---|
| hsa-miR-107 | agcagcattgtacagggctatca |
| hsa-miR-122 | Tggagtgtgacaatggtgtttg (SEQ ID No.1) |
| hsa-miR-1246 | aatggatttttggagcagg |
| hsa-miR-192 | ctgacctatgaattgacagcc |
| hsa-miR-199 | acagtagtctgcacattggtta |
| hsa-miR-21 | Tagcttatcagactgatgttga (SEQ ID No.2) |
| hsa-miR-2115 | Catcagaattcatggaggctag (SEQ ID No.3) |
| hsa-miR-221 | Agctacattgtctgctgggtttc (SEQ ID No.4) |
| hsa-miR-223 | tgtcagtttgtcaaatacccca |
| hsa-miR-26 | ttcaagtaatccaggataggct |
| hsa-miR-27 | Ttcacagtggctaagttccgc (SEQ ID No.5) |
| hsa-miR-4499 | Gattgctctgcgtgcggaatcgac (SEQ ID No.6) |
| hsa-miR-801 | gattgctgcgtgcggaatcgac |
| RNU6-1 | gtgctcgcttcggcagcacatatactaaaattggaacgatacagagaagattagcatggcccc tgcgcaa ggatgacacgcaaattcgtgaagcgttccatatttt |

### Example 4 Validation of microRNAs screened out by microRNA microassay in a training set of 292 samples

Primers (miRNA primer sequences are shown in Table 5) were designed for quantitative Real-time PCR testing of miRNAs in serum from 68 healthy controls, 62 patients with chronic hepatitis B, 61 patients with liver cirrhosis and 101 patients with liver cancer.
(1) Preparation of cDNA samples: (a) small RNA was extracted using the miRNeasy Serum/Plasma Advanced Kit (Qiagen) following the instructions provided by the manufacturer and its concentration was quantified using a Qubit 4.0 fluorimeter, (b) the small RNA was subjected to tailing using a tailing reagent (E. coli Poly(A) Polymerase, Vazyme), and (c) the tailed small RNA was subjected to reverse transcription using a reverse transcription reagent (HiScript^{®} II Reverse Transcriptase, Vazyme) to obtain cDNA.
(2) Real-time PCR: Real-time fluorescence quantitative PCR (Taq Pro U Multiple Probe qPCR MIX, Vazyme) was performed to test and compare changes in miRNA expression levels in serum samples of the control group (healthy controls, patients with chronic hepatitis B and patients with liver cirrhosis) and patients with liver cancer. The miRNA expression levels in serum samples of each group were tested using RNU6-1 as an internal reference.

The HCC group was compared to the control group using the Kruskal-Wallis test, and the HCC group was compared to the control group using the Mann-Whitney unpaired test. MicroRNA diagnostic markers were selected based on the training set using a stepwise logistic regression model, a predictive probability of HCC was used as a surrogate marker to establish a receiver operating characteristic (ROC) curve, the area under the ROC curve (AUC) was used to evaluate the diagnostic efficacy of the combination of serum AFP and microRNA, and ROC and regression analysis were performed using MedCalc (19.8) software. From the result analysis, 6 miRNAs namely hsa-miR-122, hsa-miR-21, hsa-miR-2115, hsa-miR-221, hsa-miR-27 and hsa-miR-4499 were significantly different between the control group and the patients with liver cancer.

The expression profiles and diagnostic efficacy of the 6 candidate microRNAs in the training set are shown in Table 6 in FIG. 9, where 6microRNA combination * AUC = 0.937 (0.940, 0.912)

The above miRNAs with significant expression difference were incorporated into a Logistics regression equation to obtain: *Logit (p=HCC)=-1.772+0.097*hsa-miR-122 +0.295*hsa-miR-21 -1.123*hsa-miR-2115 -0.322*hsa-miR-221 +0.606*hsa-miR-27 +0.057*hsa-miR-4499.

The AUC of the microRNA combination was significantly greater than the AUC of AFP (0.937 vs. 0.801, p<0.001, FIG. 3-A). See FIG. 3.

**Table 5 miRNA primer sequences**

| Primer name | Corresponding miRNA primer sequence (5'→3') |
|---|---|
| hsa-miR-107-F | aacaagagcagcattgtacaggg |
| hsa-miR-122-F | Gccgagtggagtgtgacaatg (seq id no.7) |
| hsa-miR-1246-F | aacacgcaatggatttttggage |
| hsa-miR-192-F | tcggcaggctgacctatgaat |
| hsa-miR-199-F | gccgagacagtagtctgcaca |
| hsa-miR-21-F | Tcggcaggtagcttatcagac (seq id no.8) |
| hsa-miR-2115-F | catcagaattcatggaggct(seq id no.9) |
| hsa-miR-221-F | Gccgagagctacattgtctgc (seq id no.10) |
| hsa-miR-223-F | tcggcaggtgtcagtttgtca |
| hsa-miR-26-F | tcggcaggttcaagtaatcca |
| hsa-miR-27-F | gccgagttcacagtggctaag (seq id no.11) |
| hsa-miR-4499-F | Gattgctctgcgtgcggaatc (seq id no.12) |
| hsa-miR-801-F | gattgctctgcgtgcggaatc |
| RNU6-1-F | aacaagcgcaaggatgacacg |
| Universal Rp | Cagtgcagggtccgaggt (seq id no.12) |
| Universal RT primer | cagtgcagggtccgaggtcagagccacctgggcaattttttttttttvn |
| Universal Taqman Probe | fam-tgcccaggtggct-mgbnfq |

### Example 5 Validation of the above microRNA combination in a validation set of 298 samples

This example is shown in FIGS. 3 and 4.

Parameters estimated from the training set were used to predict the probability of being diagnosed with HCC on an independent validation set (298 serum samples). Similarly, the quantitative RT-PCR testing method was used for the experiment. The predictive probability was used to establish the receiver operating characteristic curve.

Comparison of AUC between the microRNA combination and AFP in the validation set showed that the microRNA combination had significantly higher diagnostic accuracy than AFP (AUC: 0.938 vs. 0.747, p <0.001, FIG. 3-B).

The MicroRNA combination was also compared to AFP in the efficacy of distinguishing the HCC group from the healthy group, the CHB group and the liver cirrhosis group, as shown in FIG. 4. This analysis demonstrated that both the MicroRNA combination and AFP can distinguish HCC from non-HCC. However, the AUC of the microRNA combination was significantly greater than that of AFP (HCC vs. healthy group: 0.990 vs. 0.793, p<0.001; HCC vs. CHB: 0.887 vs. 0.734, p <0.001; HCC vs liver cirrhosis group: 0.826 vs. 0.712, p<0.001; FIGS. 4-A, 4-B and 4-C).

### Example 6 Diagnostic performance of the MicroRNA combination and AFP at different stages of BCLC

The diagnostic performance of the MicroRNA combination and AFP at different stages of BCLC was further evaluated, as shown in FIG. 5. The diagnostic performance of the MicroRNA combination was significantly higher than that of AFP in diagnosing early, intermediate and advanced HCC (BCLC stages 0, A, B and C) (BCLC stage 0, AUC 0.955 vs. 0.670, p<0.001; BCLC stage A, AUC 0.982 vs. 0.719, p<0.001; BCLC stage B, AUC 0.912 vs. 0.815, p<0.001; BCLC stage C, AUC 0.955 vs. 0.830, p<0.001; FIGS. 5-A, 5-B, 5-C and 5-D).

### Example 7 Diagnostic performance of the MicroRNA combination in normal AFP (≤ 20ng/mL) and high AFP (> 20ng/mL) groups

This Example 7 is shown in FIG. 6. The diagnostic accuracy of the microRNA combination was assessed according to AFP level. In the normal AFP (≤ 20ng/mL) group, the AUC of the microRNA combination was significantly greater than that of AFP (0.956 vs. 0.676, p<0.001, FIG. 6-A). In the high AFP (> 20ng/mL) group, the AUC of the microRNA combination was not significantly different from that of AFP (0.968 vs. 0.940, p=0.26, FIG. 6-B).

The results obtained demonstrated specific expression of microRNAs in the serum of patients with hepatocellular carcinoma. Therefore, the microRNA set specified herein can be used as a unique microRNA biomarker. Through hepatocellular carcinoma serum microRNA expression profiling, it can not only early diagnose the occurrence of liver cancer, but also distinguish hepatocellular carcinoma from chronic hepatitis B and liver cirrhosis.

The present invention provides a unique molecular marker for screening, early diagnosis, and differential diagnosis of hepatocellular carcinoma in blood samples for the identification and validation of serum microRNA expression biomarkers.

## Claims

1. Liver cancer-associated serum microRNA markers, consisting of hsa-miR-122, hsa-miR-21, hsa-miR-2115, hsa-miR-221, hsa-miR-27 and hsa-miR-4499; wherein a sequence of the hsa-miR-122 is tggagtgtgacaatggtgtttg (SEQ ID No.1), a sequence of the hsa-miR-21 is tagcttatcagactgatgttga (SEQ ID No.2), a sequence of the hsa-miR-2115 is catcagaattcatggaggctag (SEQ ID No.3), a sequence of the hsa-miR-221 is agctacattgtctgctgggtttc (SEQ ID No.4), a sequence of the hsa-miR-27 is ttcacagtggctaagttccgc (SEQ ID No.5), and a sequence of the hsa-miR-4499 is gattgctctgcgtgcggaatcgac (SEQ ID No.6).

2. A new method for diagnosing liver cancer, wherein after sampling and processing the serum to be tested, relative expression level of the liver cancer-associated serum microRNA markers in claim 1 in serum is tested using Real-time PCR method; reagents for testing the liver cancer-associated serum microRNA markers hsa-miR-122, hsa-miR-21, hsa-miR-2115, hsa-miR-221, hsa-miR-27 and hsa-miR-4499 contain primers for the markers, and specific composition is as follows: a sequence of a forward primer hsa-miR-122-F of the marker hsa-miR-122 (SEQ ID No.1) is gccgagtggagtgtgacaatg (SEQ ID No.7), a sequence of a forward primer hsa-miR-21-F of the marker hsa-miR-21 (SEQ ID No.2) is tcggcaggtagcttatcagac (SEQ ID No.8); a sequence of a forward primer hsa-miR-2115-F of the marker hsa-miR-2115 (SEQ ID No.3) is catcagaattcatggaggct (SEQ ID No.9); a sequence of a forward primer hsa-miR-221-F of the marker hsa-miR-221 (SEQ ID No.4) is gccgagagctacattgtctgc (SEQ ID No.10); a sequence of a forward primer hsa-miR-27-F of the marker hsa-miR-27 (SEQ ID No.5) is gccgagttcacagtggctaag (SEQ ID No.11); a sequence of a forward primer hsa-miR-4499-F of the marker hsa-miR-4499 (SEQ ID No.6) is gattgctctgcgtgcggaatc (SEQ ID No.12); and a sequence of a universal reverse primer (Universal Rp) of the markers is cagtgcagggtccgaggt (SEQ ID No.13).

3. The new method according to claim 2, wherein the liver cancer-associated serum microRNA markers hsa-miR-122, hsa-miR-21, hsa-miR-2115, hsa-miR-221, hsa-miR-27 and hsa-miR-4499 are tested to obtain ΔCt values which are entered into a determination formula:
comprehensive determination value = -1.772+0.097*ΔCt hsa-miR-122 +0.295*ΔCt hsa-miR-21 -1.123*ΔCt hsa-miR-2115 -0.322*ΔCt hsa-miR-221 +0.606*ΔCt hsa-miR-27 +0.057*ΔCt hsa-miR-4499;
and the comprehensive determination value ≥ 0 is considered positive and indicates a high risk of liver cancer, and the comprehensive determination value < 0 is considered negative and indicates a low risk of liver cancer.
